Europäisches Patentamt

European Patent Office

Office européen des brevets

(11) Publication number: **0 273 708 B1**

# EUROPEAN PATENT SPECIFICATION

(45) Date of publication of patent specification: 23.03.94    (51) Int. Cl.5: **C12P 7/64**, C12N 1/20,
//(C12N1/20,C12R1:38,1:01)

(21) Application number: 87311372.4

(22) Date of filing: 23.12.87

The file contains technical information submitted after the application was filed and not included in this specification

(54) **Process for production of eicosapentaenoic acid.**

(30) Priority: 26.12.86 JP 308784/86
02.02.87 JP 20510/87
02.02.87 JP 20511/87
06.03.87 JP 49932/87
02.04.87 JP 79806/87

(43) Date of publication of application:
06.07.88 Bulletin 88/27

(45) Publication of the grant of the patent:
23.03.94 Bulletin 94/12

(84) Designated Contracting States:
AT BE CH DE ES FR GB GR IT LI LU NL SE

(56) References cited:

CHEMICAL ABSTRACTS, vol. 104, no. 23, 9th June 1986, page 603, abstracts no.205535r, Columbus, Ohio, US; & JP-A-61 31 092 (IKEDA TOHKA INDUSTRY CO., LTD) 13-02-1986

CHEMICAL ABSTRACTS, vol. 105, no. 25, 22nd December 1986, page 625, abstractno. 224603y, Columbus, Ohio, US; & JP-A-61 177 990 (HARIMA CHEMICALS, INC.) 09-08-1986

(73) Proprietor: SAGAMI CHEMICAL RESEARCH CENTER
4-5, Marunouchi 1-chome
Chiyoda-ku Tokyo 100(JP)

(72) Inventor: **Yazawa, Kazunaga**
Guriin Haitsu DI-501
571 Unomori Sagamihara-shi kanagawa(JP)
Inventor: **Araki, Keiko**
1879-6, Kanamori
Machida-shi Tokyo(JP)
Inventor: **Okazaki, Noriko**
1001 Rockville Pike
Apt.No.1515
Rockville Maryland 20852(US)
Inventor: **Numao, Naganori**
1-9-2, Minamidai
Sagamihara-shi Kanagawa(JP)
Inventor: **Kondo, Kiyosi**
5-16-4, Chuorinkan
Yamato-shi Kanagawa(JP)

(74) Representative: **Sheard, Andrew Gregory et al**
Kilburn & Strode
30, John Street
London WC1N 2DD (GB)

CHEMICAL ABSTRACTS, vol. 107, no. 1, 6th July 1987, page 363, abstract no.

3911m, Columbus, Ohio, US; C.O. WIRSEN et al.: "Membrane lipids of apsychrophilic and barophilic deep-sea bacterium", & CURR. MICROBIOL.1987, 14(6), 319-22

**Description**

The present invention relates to novel processes for the production of eicosapentaenoic acid and lipid containing eicosapentaenoic acid, by fermentation and using microorganisms belonging to the genus Pseudomonas, Alteromonas or Shewanella.

Poly-unsaturated fatty acids represented by eicosapentaenoic acid are important as a component of bio-membranes in an organism. The known pharmacological actions of eicosapentaenoic acid include blood platelet coagulation-inhibition (thrombolytic action), lowering of blood level of neutral fat, lowering of blood levels of VLDL-cholesterol and LDL-cholesterol and increase of blood level of HDL-cholesterol (anti-atherosclerotic action), lowering of blood viscosity, anti-hypertension, anti-inflammation, and anti-tumor actions. Moreover, eicosapentaenoic acid acts as a substrate for the formation of the group of prostaglandins, and thus are essential for higher mammals including humans. In particular, eicosapentaenoic acid is important as a substrate for the type 3 prostaglandin, inhibits a blood platelet coagulation action, and is promising as a therapeutic and propylactic agent for thrombosis. Moreover, among poly-unsaturated fatty acids responsible for lowering the plasma cholesterol level, eicosapentaenoic acid in particular exhibits a high level of activity in lowering the plasma cholesterol level. Therefore, eicosapentaenoic acid is extremely effective in comparison with other fatty acids, such as linoleic acid, naturally present in plant oil. Eicosapentaenoic acid is also known to be an essential nutrient for fish.

An epidemiological study disclosed by Dyerberg, Am. J. Clin. Nutr. 28, 958, 1975, Denmark suggests the possibility of using eicosapentaenoic acid as a pharmaceutical agent and diet food on the basis of the anti-thrombotic action and lipid - lowering action thereof. However, as evident from the structure thereof, a chemical synthesis of eicosapentaenoic acid is very difficult, and therefore, the industrial production of eicosapentaenoic acid is difficult. Under these circumstances, an ingestion of sardine, mackerel, and saury containing a relatively large amount of eicosapentaenoic acid is recommended.

At present, the commercial eicosapentaenoic acid-containing products provided for diet foods are obtained from fish oil prepared by cooking fish, and contain eicosapentaenoic acid in an amount of 10 to 30% by weight. Fish oil prepared by the cooking process comprises a mixture of glycerides which contain various kinds of fatty acids, and therefore, the isolation of eicosapentaenoic acid from the fish oil is difficult. Moreover, since eicosapentaenoic acid is a straight-chain fatty acid with 20 carbon atoms containing five double bonds, all of which are in a cis configuration, it is unstable and susceptible to oxidation. Therefore, the isolation of eicosapentaenoic acid from fish oil must be carried out in such a manner that oxygen, light, and heat are excluded during the process. Moreover, it is difficult to eliminate organic solvents used for the isolation of eicosapentaenoic acid from the product under the usual reduced pressure. Therefore, a complete elimination of the organic solvents is a problem from the technical and economical points of view.

The eicosapentaenoic acid used for pharmaceutical purposes is produced by hydrolysing fish oil extracted by any method, with enzymes, or under an alkaline conditions, to liberate free fatty acids, and optionally, converting the fatty acids to methyl esters or ethyl esters thereof, subjecting the esters to fractional crystallization under a low temperature, urea-addition, distillation under a reduced pressure, reverse phase chromatography or the like, to prepare a product containing at least 90% eicosapentaenoic acid. However, the eicosapentaenoic acid concentrates thus prepared contain organic residual solvents used in the extraction process and have been deteriorated by intermolecular polymerization, isomerization, oxidation or the like. Moreover, eicosapentaenoic acid products produced from fish oil might contain docosenoic acids, which are suspected to be a causal substance of cardio disease, and therefore it is a problem to use them in diet food and pharmaceuticals. Finally, eicosapentaenoic acid products produced from fish oil may emit an unpleasant "fishy" odor.

Recently, to eliminate the above-mentioned disadvantages originating from the use of fish, processes for the production of eicosapentaenoic acid using microorganisms such as chlorella, phaeodactylum, euglena, or algae have been disclosed. For example, J.L. Gellerman and H. Schlenk, BBA, 573, 23, 1979, and Yamada et al, at the Conference of the Fermentation Technology Association of Japan, 1986, reported fungi which produce eicosapentaenoic acid. Such microbial processes for the production of eicosapentaenoic acid are advantageous in that isolation and purification of the target fatty acid from a microbially produced fatty acid mixture is relatively easy, and a preferential production of the target fatty acid in relation to other fatty acids is relatively simple if the fermentation process can be controlled. However, the above mentioned processes require a incubation time of as long as seven days to one month.

Moreover, Summary of the Conference of the Association of Agricultural Chemistry of Japan published on March 10, 1987 discloses production of arachidonic acid and eicosapentaenoic acid by Mortierella.

Accordingly, the present invention provides a novel process for production of eicosapentaenoic acid comprising the steps of:

a) culturing a microorganism capable of producing lipid containing eicosapentaenoic acid belonging to the genus Pseudomonas, Alteromonas or Shewanella, to produce lipid containing eicosapentaenoic acid; and

b) recovering the eicosapentaenoic acid from the cultured product.

Moreover, in a second aspect, the present invention also provided a process for production of a lipid containing eicosapentaenoic acid comprising the steps of:

a) culturing a microorganism capable of producing lipid containing eicosapentaenoic acid belonging to the genus Pseudomonas, Alteromonas or Shewanella, to produce lipid containing eicosapentaenoic acid; and

b) recovering the lipid containing eicosapentaenoic acid.

Preferred embodiments will now be described.

According to the present invention, any microorganisms capable of producing eicosapentaenoic acid and belonging to the genus Pseudomonas, Alteromonas or Shewanella, can be used.

Examples of strains belonging to Pseudomonas according to the present invention include Pseudomonas putrefaciens SCRC-2878 FERM BP-1623, which has been newly isolated and identified by the present inventors.

Examples of strains belonging to Alteromonas are Alteromonas putrefaciens, such as Alteromonas putrefaciens SCRC-2871 FERM-BP 1624 and Alteromonas putrefaciens subspecies sagamifaciens SCRC-1162 FERM-BP 1626, which have been newly isolated and identified by the present inventors. Moreover, known strains of Alteromonas putrefaciens IAM-1510, and IAM-12425 are available from the Institute of Applied Microbiology, University of Tokyo, Yayoi I-I-I, Bunkyo-ku, Tokyo, Japan 113 (IAM), and can be used for the production of eicosapentaenoic acid. Further, microorganisms belonging to Alteromonas are Alteromonas hanedai, such as Alteromonas hanedai IAM-12641, which is also available from the IAM.

A microorganism capable of producing eicosapentaenoic acid and belonging to Shewanella is, for example, Shewanella putrefaciens SCRC-2874 FERM-BP 1625, which has been newly isolated and identified by the present inventor.

The above-mentioned new strains were isolated according to the following procedure. Namely, the plate medium of a composition set forth in Table 1 was prepared.

Table 1

| Meat extract | 1% |
|---|---|
| Peptone | 1% |
| NaCl | 0.5% |
| Agar | 1.5% |
| Water | |
| (pH 7.0) | |

To this agar medium, a sample obtained from various marine sources, which had been appropriately diluted with physiological saline, was inoculated, and the agar plate was incubated at 25°C for one or two days to develop microbial colonies. The colonies were picked up to slant media having the same composition as described above. In this manner, many strains were isolated from samples obtained from various marine areas.

A medium with the same composition as described above, except that the agar was omitted, was distributed to test tubes in an amount of 5 ml per tube, and autoclaved. The strains isolated as described above were inoculated to the test tubes, which were then incubated at 25°C for one or two days. The cultures were then assayed to determine the content of eicosapentaenoic acid. The origins of the isolated strains are shown in Table 2.

Table 2

| Strains | Origin |
|---|---|
| SCRC-2871, SCRC-2874, SCRC-1162, SCRC-2878 | Sagami Bay |

The taxonomical properties of the above-mentioned strains are shown in Table 3.

Table 3 (Continued)

| Observation | SCRC-2878 |
|---|---|
| a) Morphology | |
| 1 Shape of cell | short rod |
|    Size | 1.0 x 2.0 μm |
| 2 Pleomorphism | none |
| 3 Motility | + |
|    Flagella | single polar |
| 4 Sporulation | − |
| 5 Gram stain | − |
| 6 Acid-fastness | − |
| b) Growth characteristics | |
| 1 Bouillon agar plate (at 25°C, for 2 days) | |
|    Size of colony | 2.0 − 2.4 mm |
|    Shape of colony | round |
|    Surface of colony | smooth |
|    Elevation of colony | raised |
|    Edge of colony | entire |
|    Color of colony | light yellow |

Table 3 (Continued)

| | Observation | SCRC-2878 |
|---|---|---|
| c) | Physiological properties | |
| 1 | Nitrate reduction | + |
| 2 | Denitrification | − |
| 3 | Methylred test | − |
| 4 | Voges-Proskauer test | − |
| 5 | Indole production | − |
| 6 | Hydrogen sulfide formation | + |
| 7 | Hydrolysis of starch | − |
| 8 | Citrate utilization | |
| | Koser | + |
| | Christensen | + |
| 9 | Nitrate utilization | + |
| 10 | Pigment formation | |
| | King A medium | − |
| | King B medium | − |
| 11 | Urease | − |

Table 3 (Continued)

| | Observation | SCRC-2878 |
|---|---|---|
| | Transparency of colony | opaque |
| | Gloss of colony | dull |
| | Formation of soluble pigment | − |
| 2 | Bouillon agar slant (at 25°C, for 2 days) | |
| | Growth | abundant |
| | Gloss of colony | dull |
| 3 | Bouillon liquid (at 25°C, for 2 days) | |
| | Growth at surface | none |
| | Turbidity | turbid |
| | Precipitation | powdery |
| | Gas formation | none |
| 4 | Bouillon gelatin (at 25°C, for 2 days) | |
| | Gelatin liquefaction | liquefied |
| | Litmus milk | no coagulation |
| | | change to yellow |

EP 0 273 708 B1

Table 3 (Continued)

| Observation | SCRC-2878 |
|---|---|
| 9. Lactose | − |
| 10. Trehalose | − |
| 11. D-Sorbitol | − |
| 12. D-Mannitol | − |
| 13. Inositol | − |
| 14. Glycerol | − |
| 15. Starch<br>(No gas production in each case) | − |
| d) Other properties | |
| Growth on SS agar medium | + |
| Growth on MacConkey agar medium | + |
| Growth on 6.5% NaCl | + |
| DNase | + |
| Ornithine decarboxylase | + |
| Arginine dehydrolation | − |
| Gelatin liquefaction | + |

Table 3 (Continued)

| Observation | SCRC-2878 |
|---|---|
| 12 Oxidase | + |
| 13 Catalase | + |
| 14 Growth range | |
| pH | 6 − 9 |
| Temperature | 5 − 30°C |
| 15 Oxygen requirement | aerobic |
| 16 O-F test (glucose) | − |
| 17 Acid/gas formation from sugar | |
| 1. L-Arabinose | − |
| 2. D-Xylose | − |
| 3. D-Glucose | + |
| 4. D-Mannose | − |
| 5. D-Fructose | + |
| 6. D-Galactose | − |
| 7. Maltose | + |
| 8. Sucrose | + |

EP 0 273 708 B1

Table 3 (Continued)

| Observation | SCRC-2871 | SCRC-2874 | SCRC-1162 |
|---|---|---|---|
| a) Morphology | | | |
| 1 Shape of cell | short rod | short rod | short rod |
| Size | 1.0 x 2.0 μm | 1.0 x 2.0 μm | 1.0 x 2.0 μm |
| 2 Pleomorphism | none | none | none |
| 3 Motility | + | + | + |
| Flagella | single polar | single polar | peritrichous |
| 4 Sporulation | − | − | − |
| 5 Gram stain | − | − | − |
| 6 Acid-fastness | − | − | − |
| b) Growth characteristics | | | |
| 1 Bouillon agar plate (at 25°C, for 2 days) | | | |
| Size of colony | 2.0 − 2.4 mm | 2.0 x 2.4 mm | 0.6 − 0.8 mm |
| Shape of colony | round | round | round |
| Surface of colony | smooth | smooth | smooth |
| Elevation of colony | raised | raised | raised |
| Edge of colony | entire | entire | entire |
| Color of colony | light yellow | light yellow | light yellow |

EP 0 273 708 B1

Table 3 (Continued)

| Observation | SCRC-2871 | SCRC-2874 | SCRC-1162 |
|---|---|---|---|
| Transparency of colony | opaque | opaque | translucent |
| Gloss of colony | dull | dull | dull |
| Formation of soluble pigment | – | – | – |
| 2 Bouillon agar slant (at 25°C, for 2 days) | | | |
| Growth | abundant | abundant | abundant |
| Gloss of colony | dull | dull | dull |
| 3 Bouillon liquid (at 25°C, for 2 days) | | | |
| Growth at surface | none | none | none |
| Turbidity | turbid | turbid | turbid |
| Precipitation | powdery | powdery | powdery |
| Gas formation | none | none | none |
| 4 Bouillon gelatin (at 25°C, for 2 days) | | | |
| Gelatin liquefaction | liquefied | liquefied | liquefied |
| Litmus milk | no coagula-tion | no coagula-tion | no change |
| | change to yellow | change to yellow | |

EP 0 273 708 B1

Table 3 (Continued)

| Observation | | SCRC-2871 | SCRC-2874 | SCRC-1162 |
|---|---|:---:|:---:|:---:|
| c) | Physiological properties | | | |
| 1 | Nitrate reduction | + | + | + |
| 2 | Denitrification | − | − | − |
| 3 | Methylred test | − | − | − |
| 4 | Voges-Proskauer test | − | − | − |
| 5 | Indole production | − | − | − |
| 6 | Hydrogen sulfide formation | + | + | + |
| 7 | Hydrolysis of starch | − | − | − |
| 8 | Citrate utilization | | | |
| | Koser | + | + | − |
| | Christensen | + | + | − |
| 9 | Nitrate utilization | + | + | − |
| 10 | Pigment formation | | | |
| | King A medium | − | − | − |
| | King B medium | − | − | − |
| 11 | Urease | − | − | + |

EP 0 273 708 B1

Table 3 (Continued)

| | Observation | SCRC-2871 | SCRC-2874 | SCRC-1162 |
|---|---|---|---|---|
| 12 | Oxidase | + | + | + |
| 13 | Catalase | + | + | + |
| 14 | Growth range | | | |
| | pH | 6 – 9 | 6 – 9 | 6 – 9 |
| | Temperature | 5 – 30°C | 5 – 30°C | 5 – 30°C |
| 15 | Oxygen requirement | aerobic | aerobic | aerobic |
| 16 | O-F test (glucose) | – | – | – |
| 17 | Acid/gas formation from sugar | | | |
| | 1. L-Arabinose | – | – | – |
| | 2. D-Xylose | – | – | – |
| | 3. D-Glucose | + | + | + |
| | 4. D-Mannose | – | – | – |
| | 5. D-Fructose | + | + | – |
| | 6. D-Galactose | – | – | – |
| | 7. Maltose | + | + | – |
| | 8. Sucrose | + | + | – |

Table 3 (Continued)

| Observation | SCRC-2871 | SCRC-2874 | SCRC-1162 |
|---|---|---|---|
| 9. Lactose | − | − | − |
| 10. Trehalose | − | − | − |
| 11. D-Sorbitol | − | − | − |
| 12. D-Mannitol | − | − | − |
| 13. Inositol | − | − | − |
| 14. Glycerin | − | − | − |
| 15. Starch<br>(No gas production in each case) | − | − | − |
| d)    Other properties | | | |
| Growth on SS agar medium | + | + | + |
| Growth on MacConkey agar medium | + | + | + |
| Growth on 6.5% NaCl | + | + | + |
| DNase | + | + | + |
| Ornithine decarboxylase | + | + | − |
| Arginine dehydrolation | − | − | − |
| Gelatin liquefaction | + | + | |
| G+C content of DNA | 47.1% | 46.9% | 44.0% |

## Table 3 (Continued)

| Observation | SCRC-2871 | SCRC-2874 | SCRC-1162 |
|---|---|---|---|
| $Na^+$ requirement | | | + |
| Quinone type | | | |
| Ubiquinone 7 | | + | + |
| Ubiquinone 8 | | + | + |
| Menaquinone | | + | + |
| Methylmenaquinone | | + | + |

On the basis of the above-mentioned taxonomical properties, and according to criteria described in Bergey's Manual of Determinative Bacteriology, Eighth Ed., 1974 (Reference 1); Manual of Clinical Microbiology, Third Ed., 1980 (Reference 2); Bergey's Manual of Systematic Bacteriology Volume 1, 352, 1984 (Reference 3); and Journal of General Microbiology 129, 3057-3074, 1983 (Reference 4), the above-mentioned strains were identified as follows:

Since SCRC 2878 bacteria are gram negative rod bacteria which are aerobic, motile, have a single polar flagellum, and exhibit catalase and oxidase activities, according to References 1 and 2, they belong to the genus Pseudomonas. These references describe Pseudomonas putrefaciens as a species of Pseudomonas, and the properties of this strain substantially correspond to those described in the references. Therefore, the above-mentioned strain is identified as Pseudomonas putrefaciens. Note, although some properties relating to the production of acids and gas from sugars are not identical to those described in the References, since such, properties are not important from the taxonomical point of view, and such properties generally vary within the same species, then such differences have no affect on the above-mentioned identification.

Note, according to the reference 4, however, on the basis of the G + C content in their DNAs, Pseudomonas putrefaciens is described as Alteromonas putrefaciens. This is also referred to in reference 3. More recently, M.T. MacDonell and R.R. Colwell, Systematic and Applied Microbiology 6, 171-182 (1985) proposed a new genus Shewanella on the basis of a ribonucleotide sequence of 5S rRNA, and suggested that it be designated Shewanella putrefaciens.

Since SCRC-2871 is a gram negative rod bacterium which is aerobic, motile, has a single polar flagellum, and exhibits catalase and oxidase activities, and moreover, has a G + C content in their DNAs of 47.1%, according to the above-mentioned references 1, 2, 3, and 4, it belongs to the genus Alteromonas. As a strain belonging to Alteromonas, Alteromonas putrefaciens is known, and the above-mentioned taxonomical properties of this strain substantially correspond to those described in the above-mentioned references. Therefore, this strain is identified as Alteromonas putrefaciens.

Since SCRC-2874 is a gram negative rod bacterium which is aerobic, motile, has a single polar flagellum, and exhibits catalase and oxidase activities, then according to the references 1 and 2, it belongs to Pseudomonas putrefaciens. According to the reference 4, however, on the basis of the G + C content in their DNAs, Pseudomonas putrefaciens is described as Alteromonas putrefaciens. This is also referred to in reference 3. More recently, M.T. MacDonell and R.R. Colwell, Systematic and Applied Microbiology 6, 171-182 (1985) proposed a new genus Shewanella on the basis of a ribonucleotide sequence of 5S rRNA, and suggested that it be designated Shewanella putrefaciens. Therefore, taking this into consideration, SCRC-2874 is identified as Shewanella putrefaciens.

SCRC-1162 has the following properties: (1) gram negative, (2) motile, (3) non-sporulation and aerobic rod, (4) O-F test negative, (5) catalase and oxidase positive, (6) nitrate reduction positive, (7) hydrogen sulfide formation positive, (8) gelatin liquefaction and DNA hydrolysis positive, (9) acid formation ( + ) and gas formation (-) from glucose, (10) quinone type: ubiquinones 7 and 8, menaquinone and methylmenaquinone, (11) peritrichous flagella, and (12) $Na^+$ requirement. On the basis of the above-mentioned properties, according to the References 1, 2, 3, and 4, the strain SCRC-1162 is identified as follows: From the properties (1) to (10), the strain could be considered to be an Alteromonas putrefaciens related strain, but since it has peritrichous flagella as observed by electron microscope, does not have an ornithine decarboxylase activity, and requires $Na^+$, the present inventor decided that this strain should belong to a new subspecies, and designated and identified it as Alteromonas putrefaciens subspecies sagamifaciens.

The above-mentioned microorganisms were deposited with the Fermentation Research Institute Agency of Industrial Science and Technology, Ministry of International Trade and Industry, 1-1-3 Yatabe-machi Higashi, Tsukuba-shi, Ibaraki-ken, Japan under FERM P-Numbers, and transferred to international deposition under the Deposit of Microorganisms for the Purposes of Patent Procedure (Budapest Treaty) under FERM BP-Numbers as follows:

Pseudomonas putrefaciens SCRC-2878
December 26, 1986 FERM P-9114
December 17, 1987 FERM BP-1623
Alteromonas putrefaciens SCRC-2871
January 28, 1987 FERM P-9158
December 17, 1987 FERM BP-1624
Shewanella putrefaciens SCRC-2874
January 28, 1987 FERM P-9159
December 17, 1987 FERM BP-1625
Alteromonas putrefaciens subspecies sagamifaciens SCRC-1162
February 20, 1987 FERM P-9210
December 17, 1987 FERM BP-1626

The above-mentioned strains for the present invention may be converted to strains having an eicosapentaenoic acid productivity higher than the original strain, by conventional mutation and selection procedures.

The microorganisms used in the present invention can be preserved by a conventional method, such as on an agar slant medium or by lyophilization. As the agar slant medium, a conventional medium for the preservation of microorganism belonging the genera Pseudomonas, Alteromonas or Shewanella, for example, the above-mentioned medium, can be used. Lyophilization also may be carried out by a conventional method.

For the production of eicosapentaenoic acid, one of the above-mentioned microorganism is cultured in a medium. The medium may be any medium in which the microorganism used can grow and produce eicosapentaenoic acid. The medium contains, as a nitrogen source, for example, yeast extract, peptone or meat extract, or a combination thereof. Moreover, the medium may contain, as a carbon source, various kinds of sugar such as glucose, fructose, maltose, and sucrose; organic acids, such as intermediates of the TCA cycle such as citrate, fumarate and pyruvate, other organic acids such as maleate and acetate; and amino acids such as aspartate and glutamate. The medium preferably contains sodium chloride, or sea water or artificial sea water. Note, Alteromonas hanedai, and Alteromonas putrefaciens subspecies sagamifaciens require sodium chloride.

The microorganisms of the present invention can be cultured in a liquid medium or on a solid medium. The pH value of the medium is 6 to 9, preferably 7 to 8. To obtain a large amount of the target product eicosapentaenoic acid, the microorganism is cultured in a liquid medium by stationary culture, or more preferably, by a shaking culture or aeration-agitation culture, under aerobic conditions. The microorganism is cultured at any temperature at which the microorganism can grow and produce eicosapentaenoic acid. The temperature is preferably 5°C to 30°C, and more preferably, 15°C to 25°C. For Alteromonas hanedai, the temperature is preferably 5°C to 25°C, and more preferably, 10°C to 20°C. Culturing is carried out for a period sufficient to produce eicosapentaenoic acid in an amount enough to enable the recovery thereof, preferably for 8 to 48 hours.

Next, eicosapentaenoic acid is recoverable from the culture by a conventional procedure used for the isolation and purification of a fatty acid from a culture. For example, the culture is centrifuged or filtered to obtain microbial cells, which are then optionally washed with water, saline or a buffer such as a phosphate buffer. The cells are then resuspended in such a liquid to prepare a cell suspension. Next, the suspension is extracted with a conventional solvent, for example, a mixture of methanol and chloroform. The chloroform phase is then separated and evaporated to obtain a lipid material containing eicosapentaenoic acid. The lipid is then saponified to obtain free eicosapentaenoic acid or a salt thereof.

According to the present invention, a large amount of pure eicosapentaenoic acid and a lipid containing eicosapentaenoic acid can be easily produced in a short time by fermentation.

Examples

The present invention will now be further illustrated by, but is by no means limited to, the following examples.

Example 1. Production of eicosapentaenoic acid and lipid containing same by Pseudomonas putrefaciens SCRC-2878

First, 20 ℓ of a medium containing 1.0% of meat extract, 1.0% of peptone and 0.5% of NaCl (pH 7.0) was filled in a jar fermenter, and after sterilization at 121°C for 15 minutes, an inoculum of Pseudomonas putrefaciens SCRC-2878 (FERM BP-1623) was inoculated to the medium. Culturing was carried out at 25°C at pH 6.8 to 7.2 for 24 hours under an aerobic condition by aeration and agitation. After the culturing, the culture was centrifuged for 20 minutes at 6000 x G to obtain about 150 g of wet cells (corresponding to 16.5 g of dry cells). The cells were washed with 0.85% saline and resuspended in 1 ℓ of the same saline. The suspension was extracted with 1 ℓ of a mixture of chloroform and methanol (2:1 v/v), and the whole was centrifuged to obtain a chloroform phase. To the residual aqueous phase containing cells was added 600 ml of chloroform, and the whole was thoroughly shaken and centrifuged to obtain a chloroform phase. The chloroform phases were combined, and the combined chloroform phase was concentrated to dryness to obtain 1.16 g of a lipid fraction (corresponding to 7.03 g/100 g dry cells). The lipid fraction contained eicosapentaenoic acid. The lipid was saponified in 95% ethanol containing 0.3N NaOH at 80°C for one hour, to obtain a saponification product containing sodium eicosapentaenoate. The product was then neutralized with 6N HCl to obtain a product containing free eicosapentaenoic acid. Next, the neutralized product was treated with diazomethane to convert free eicosapentaenoic acid to a methyl ester thereof, and the methyl ester-containing product was analyzed by gas chromatography. As a result, it was found to contain 0.114 g (9.8 g/100 g lipid fraction, and 0.69 g/100 g dry cells) of eicosapentaenoic acid.

Example 2. Production of eicosapentaenoic acid and lipid containing same by Alteromonas putrefaciens SCRC-2871

First, 20 ℓ of a medium containing 1.0% of meat extract, 1.0% of peptone and 0.5% of NaCl (pH 7.0) was filled in a jar fermenter, and after sterilization at 121°C for 15 minutes, an inoculum of Alteromonas putrefaciens SCRC-2871 (FERM BP-1624) was inoculated to the medium. Culturing was carried out at 25°C at pH 6.8 to 7.2 for 24 hours under an aerobic condition by aeration and agitation. After the culturing, the cultured broth was centrifuged for 20 mi-nutes at 6,000 x G to obtain about 135 g of wet cells (corresponding to 15.0 g of dry cells). The cells were washed with 0.85% saline and resuspended in 1 ℓ of the same saline. The suspension was extracted with 1 ℓ of a mixture of chloroform and methanol (2:1 v/v), and the whole was centrifuged to obtain a chloroform phase. To the residual aqueous phase containing cells was added 600 ml of chloroform, and the whole was thoroughly shaken and centrifuged to obtain the chloroform phase. The chloroform phases were combined, and the combined chloroform phase was concentrated to dryness to obtain 1.05 g of lipid fraction (corresponding to 7.00 g/100 g dry cells). The lipid fraction contained eicosapentaenoic acid. The lipid was saponified in 95% ethanol containing 0.3N NaOH at 80°C for one hour to obtain a saponification product containing sodium eicosapentaenoate. The product was then neutralized with 6N HCl to obtain a product containing free eicosapentaenoic acid. Next, the neutralized product was treated with diazomethane to convert free eicosapentaenoic acid to a methyl ester thereof, and the methyl ester-containing product was analyzed by gas chromatography. As a result, it was found to contain 0.111 g (10.6 g/100 g lipid fraction, 0.74 g/100 g dry cells) of eicosapentaenoic acid.

The above-mentioned free eicosapentaenoic acid containing product was subjected to silica gel column chromatography using n-hexane/ethyl ether (9:1) as an eluate to obtain a fraction containing purified eicosapentaenoic acid, which fraction was evaporated to dryness to obtain 0.10 g of eicosapentaenoic acid.

Example 3. Production of eicosapentaenoic acid and lipid containing same by Shewanella putrefaciens SCRC-2874

First, 20 ℓ of a medium containing 1.0% of meat extract, 1.0% of peptone and 0.5% of NaCl (pH 7.0) was filled in a jar fermenter, and after sterilization at 121°C for 15 minutes, an inoculum of Shewanella putrefaciens SCRC-2874 (FERM BP-1625) was inoculated to the medium. Culturing was carried out at 25°C at pH 6.8 to 7.2 for 24 hours under an aerobic condition by aeration and agitation. After the culturing, the culture was centrifuged for 20 minutes at 6000 x G to obtain about 200 g of wet cells (corresponding to 22.5 g of dry cells). The cells were washed with 0.85% saline, and resuspended in 1 ℓ of the same saline. The suspension was extracted with 1 ℓ of a mixture of chloroform and methanol (2:1 v/v), and the whole was centrifuged to obtain a chloroform phase. To the residual aqueous phase containing cells was added 600 ml of chloroform, and the whole was thoroughly shaken and centrifuged to obtain a chloroform phase. The chloroform phases were combined, and the combined chloroform phase was concentrated to dryness to obtain 1.78 g of lipid fraction (corresponding to 7.91 g/100 g dry cells). The lipid fraction contained eicosapentaenoic acid. The lipid was saponified in 95% ethanol containing 0.3N NaOH at 80°C for one hour to obtain a saponification product containing sodium eicosapentaenoate. The product was then neutralized with 6N HCl to obtain a product containing free eicosapentaenoic acid. Next, the neutralized product was treated with diazomethane to convert free eicosapentaenoic acid to the methyl ester thereof, and the methyl ester-containing product was analyzed by gas chromatography. As a result, it was found to contain 0.241 g (13.5 g/100 g lipid fraction, 1.07 g/100 g dry cells) of eicosapentaenoic acid.

The above-mentioned free eicosapentaenoic acid containing product was subjected to silica gel column chromatography using n-hexane/ethyl ether (9:1) as an eluate to obtain a fraction containing purified eicosapentaenoic acid, which fraction was evaporated to dryness to obtain 0.22 g of eicosapentaenoic acid.

Example 4. Production of eicosap entaenoic acid and lipid containing same by Alteromonas putrefaciens subspecies sagamifaciens SCRC-1162

First, 30 ℓ of a medium containing 1.0% of meat extract, 1.0% of peptone and 0.5% of NaCl (pH 7.0) was filled in a jar fermenter, and after sterilization at 121°C for 15 minutes, an inoculum of Alteromonas putrefaciens subspecies sagamifaciens SCRC-1162 (FERM BP-1626) was inoculated to the medium. Culturing was carried out at 25°C at pH 6.8 to 7.2 for 24 hours under an aerobic condition by aeration and agitation. After the culturing, the culture was centrifuged for 20 minutes at 6000 x G to obtain about 205 g of wet cells (corresponding to 25.6 g of dry cells). The cells were washed with 0.85% saline, and resuspended in 1.5 ℓ of the same saline. The suspension was extracted with 1.5 ℓ of a mixture of chloroform and methanol (2:1 v/v), and the whole was centrifuged to obtain a chloroform phase. To the residual aqueous phase containing cells was added 900 ml of chloroform, and the whole was thoroughly shaken and centrifuged to obtain a chloroform phase. The chloroform phases were combined, and the combined chloroform phase was concentrated to dryness to obtain 2.13 g of lipid fraction (corresponding to 8.32 g/100 g dry cells). The lipid fraction contained eicosapentaenoic acid. The lipid was saponified in 95% ethanol containing 0.3N NaOH at 80°C for one hour to obtain a saponification product containing sodium eicosapentaenoate. The product was then neutralized with 6N HCl to obtain a product containing free eicosapentaenoic acid. Next, the neutralized product was treated with diazomethane to convert free eicosapentaenoic acid to the methyl ester thereof, and the methyl ester-containing product was analyzed by gas chromatography. As a result, it was found to contain 0.205 g (9.6 g/100 g lipid fraction, 0.80 g/100 g dry cells) of eicosapentaenoic acid.

The above-mentioned free eicosapentaenoic acid containing product was subjected to reverse phase silica gel column chromatography using methanol as an eluate to obtain a fraction containing purified eicosapentaenoic acid, which fraction was evaporated to dryness to obtain 0.185 g of eicosapentaenoic acid.

Example 5. Production of eicosapentaenoic acid and lipid containing same by Alteromonas hanedai IAM-12641

First, 20 ℓ of a medium containing 1.0% of meat extract, 1.0% of peptone and 0.5% of NaCl (pH 7.0) was filled in a jar fermenter, and after sterilization at 121°C for 15 minutes, an inoculum of Alteromonas hanedai IAM-12641 was inoculated to the medium. Culturing was carried out at 15°C at pH 6.8 to 7.2 for 24 hours under an aerobic condition by aeration and agitation. After the culturing, the culture was centrifuged for 20 minutes at 6000 x G to obtain about 150 g of wet cells (corresponding to 14.9 g of dry cells). The cells were washed with 0.85% saline, and resuspended in 1 ℓ of the same saline. The suspension was extracted with 1 ℓ of a mixture of chloroform and methanol (2:1 v/v), and the whole was centrifuged to obtain a chloroform phase. To the residual aqueous phase containing cells was added 600 ml of chloroform, and the whole was thoroughly shaken and centrifuged to obtain a chloroform phase. The chloroform phases were combined, and the combined chloroform phase was concentrated to dryness to obtain 1.18 g of lipid fraction (corresponding to 7.92 g/100 g dry cells). The lipid fraction contained eicosapentaenoic acid. The lipid was saponified in 95% ethanol containing 0.3N NaOH at 80°C for one hour to obtain a saponification product containing sodium eicospentaenoate. The product was then neutralized with 6N HCl to obtain a product containing free eicosapentaenoic acid. Next, the neutralized product was treated with diazomethane to convert free eicosapentaenoic acid to the methyl ester thereof, and the methyl ester-containing product was analyzed by gas chromatography. As a result, it was found to contain 0.145 g (12.3 g/100 g lipid fraction, 0.97 g/100 g dry cells) of eicosapentaenoic acid.

The above-mentioned free eicosapentaenoic acid containing product was subjected to reverse phase silica gel column chromatography using methanol as an eluate to obtain a fraction containing purified eicosapentaenoic acid, which fraction was evaporated to dryness to obtain 0.126 g of eicosapentaenoic acid.

**Claims**

1. A process for production of eicosapentaenoic acid comprising the steps of:
   a) culturing a microorganism capable of producing lipid containing eicosapentaenoic acid belonging to the genus Pseudomonas, Alteromonas or Shewanella, to produce lipid containing eicosapentaenoic acid; and
   b) recovering the eicosapentaenoic acid from the cultured product.

2. A process for production of a lipid containing eicosapentaenoic acid comprising the steps of:
   a) culturing a microorganism capable of producing lipid containing eicosapentaenoic acid belonging to the genus Pseudomonas, Alteromonas or Shewanella, to produce lipid containing eicosapentaenoic acid; and
   b) recovering the lipid containing eicosapentaenoic acid.

3. A process according to claim 1 or 2, wherein the microorganism belonging to the genus Pseudomonas is Pseudomonas putrefaciens SCRC-2878 (FERM BP-1623).

4. A process according to claim 1 or 2, wherein the microorganism belonging to the genus Alteromonas is Alteromonas putrefaciens subspecies sagamifaciens SCRC-1162 (FERM BP-1626) Alteromonas putrefaciens SCRC-2871 (FERM BP-1624), Alteromonas putrefaciens IAM-1510 and IAM-12425 or Alteromonas hanedai IAM-12641.

5. A process according to claim 1 or 2, wherein the microorganism belonging to the genus Shewanella is Shewanella putrefaciens SCRC-2874 (FERM BP-1625).

**6.** A microorganism capable of producing lipid containing eicosapentaenoic acid belonging to the genus Pseudomonas, Alteromonas, and Shewanella.

**7.** A microorganism according to claim 6, wherein the microorganism belonging to the genus Pseudomonas is <u>Pseudomonas</u> <u>putrefaciens</u> SCRC-2878 (FERM BP-1623).

**8.** A microorganism according to claim 6, wherein the microorganism belonging to the genus Alteromonas is <u>Alteromonas</u> <u>putrefaciens</u> subspecies <u>sagamifaciens</u> SCRC-1162 (FERM BP-1626) or <u>Alteromonas</u> <u>putrefaciens</u> SCRC-2871 (FERM BP-1624).

**9.** A microorganism according to claim 6, wherein the microorganism belonging to the genus Shewanella is <u>Shewanella</u> <u>putrefaciens</u> SCRC-2874 (FERM BP-1625).

**10.** A process as claimed in claim 1, wherein the microorganism is derived from a microorganism as defined in claim 3, 4 or 5.

**11.** A microorganism as claimed in claim 6, which is derived from a microorganism as defined in claim 7, 8 or 9.

**Patentansprüche**

**1.** Ein Verfahren zur Herstellung von Eicosapentaensäure, das die Schritte umfaßt:
a) Kultivieren eines Mikroorganismus, der in der Lage ist, ein Lipid, das Eicosapentaensäure enthält, herzustellen, und der zu der Gattung Pseudomonas, Alteromonas oder Shewanella gehört, um ein Lipid, das Eicosapentaensäure enthält, herzustellen; und
b) Gewinnen der Eicosapentaensäure aus dem kultivierten Produkt.

**2.** Ein Verfahren zur Herstellung eines Lipids, das Eicosapentaensäure enthält, das die Schritte umfaßt:
a) Kultivieren eines Mikroorganismus, der in der Lage ist, ein Lipid, das Eicosapentaensäure enthält, herzustellen, und der zu der Gattung Pseudomonas, Alteromonas oder Shewanella gehört, um ein Lipid, das Eicosapentaensäure enthält, herzustellen; und
b) Gewinnen des Lipids, das Eicosapentaensäure enthält.

**3.** Ein Verfahren nach Anspruch 1 oder 2, wobei der Mikroorganismus, der zu der Gattung Pseudomonas gehört, <u>Pseudomonas</u> <u>putrefaciens</u> SCRC-2878 (FERM-BP-1623) ist.

**4.** Ein Verfahren nach Anspruch 1 oder 2, wobei der Mikroorganismus, der zu der Gattung Alteromonas gehört, <u>Alteromonas</u> <u>putrefaciens</u> sub-spezies <u>sagamifaciens</u> SCRC-1162 (FERM-BP-1626), <u>Alteromonas</u> <u>putrefaciens</u> SCRC-2871 (Ferm-BP-1624), <u>Alteromonas</u> <u>putrefaciens</u> IAM-1510 und IAM-12425 oder <u>Alteromonas</u> <u>hanedai</u> IAM-12641 ist.

**5.** Ein Verfahren nach Anspruch 1 oder 2, wobei der Mikroorganismus, der zu der Gattung Shewanella gehört, <u>Shewanella</u> <u>putrefaciens</u> SCRC-2874 (FERM-BP-1625) ist.

**6.** Ein Mikroorganismus, der in der Lage ist, ein Lipid, das Eicosapentaensäure enthält, herzustellen, und der zu der Gattung Pseudomonas, Alteromonas und Shewanella gehört.

**7.** Ein Mikroorganismus nach Anspruch 6, wobei der Mikroorganismus, der zu der Gattung Pseudomonas gehört, <u>Pseudomonas</u> <u>putrefaciens</u> SCRC-2878 (FERM-BP-1623) ist.

**8.** Ein Mikroorganismus nach Anspruch 6, wobei der Mikroorganismus, der zu der Gattung Alteromonas gehört, <u>Alteromonas</u> <u>putrefaciens</u> sub-spezies <u>sagamifaciens</u> SCRC-1162 (FERM-BP-1626) oder <u>Alteromonas</u> <u>putrefaciens</u> SCRC-2871 (Ferm-BP-1624) ist.

**9.** Ein Mikroorganismus nach Anspruch 6, wobei der Mikroorganismus, der zu der Gattung Shewanella gehört, <u>Shewanella</u> <u>putrefaciens</u> SCRC-2874 (FERM-BP-1625) ist.

EP 0 273 708 B1

**10.** Ein Verfahren wie in Anspruch 1 beansprucht, wobei der Mikroorganismus von einem wie in Anspruch 3, 4 oder 5 definierten Mikroorganismus abgeleitet ist.

**11.** Ein Mikroorganismus wie in Anspruch 6 beansprucht, der von einem wie in Anspruch 7, 8 oder 9 definierten Mikroorganismus abgeleitet ist.

**Revendications**

**1.** Un procédé de préparation d'acide eicosapentaénoïque comprenant les étapes suivantes:
a) on cultive un micro-organisme capable de produire un lipide contenant de l'acide eicosapentaénoïque et appartenant au genre Pseudomonas, Alteromonas ou Shewanella pour produire un lipide contenant de l'acide eicosapentaénoïque; et
b) on récupère l'acide eicosapentaénoïque du produit mis en culture.

**2.** Un procédé de préparation d'un lipide contenant de l'acide eicosapentaénoïque comprenant les étapes suivantes:
a) on cultive un micro-organisme capable de produire un lipide contenant de l'acide eicosapentaénoïque et appartenant au genre Pseudomonas, Alteromonas ou Shewanella pour produire un lipide contenant de l'acide eicosapentaénoïque; et
b) on récupère le lipide contenant l'acide eicosapentaénoïque.

**3.** Un procédé selon la revendication 1 ou 2, caractérisé en ce que le micro-organisme appartenant au genre Pseudomonas est Pseudomonas putrefaciens SCRC-2878, FERM BP-1623.

**4.** Un procédé selon la revendication 1 ou 2, caractérisé en ce que le micro-organisme appartenant au genre Alteromonas correspond aux sous-espèces d'Alteromonas putrefaciens, sous-espèce sagamifaciens SCRC-1162 (FERM BP-1626), Alteromonas putrefaciens SCRC-2871 (FERM BP-1624), Alteromonas putrefaciens IAM-1510 et IAM-12425 ou Alteromonas hanedai IAM-12641.

**5.** Un procédé selon la revendication 1 ou 2, caractérisé en ce que le micro-organisme appartenant au genre Shewanella est Shewanella putrefaciens SCRC-2874 (FERM BP-1625).

**6.** Un micro-organisme capable de produire un lipide contenant de l'acide eicosapentaénoïque et appartenant aux genres Pseudomonas, Alteromonas et Shewanella.

**7.** Un micro-organisme selon la revendication 6, caractérisé en ce que le micro-organisme appartenant au genre Pseudomonas est Pseudomonas putrefaciens SCRC-2878 (FERM BP-1623).

**8.** Un micro-organisme selon la revendication 6, caractérisé en ce que le micro-organisme appartenant au genre Alteromonas correspond à des sous-espèces d'Alteromonas putrefaciens sagamifaciens SCRC-1162 (FERM BP-1626) ou Alteromonas putrefaciens SCRC-2871 (FERM BP-1624).

**9.** Un micro-organisme selon la revendication 6, caractérisé en ce que le micro-organisme appartenant au genre Shewanella est Shewanella putrefaciens SCRC-2874 (FERM BP- 1625).

**10.** Un procédé selon la revendication 1, caractérisé en ce que le micro-organisme provient d'un micro-organisme tel que défini dans les revendications 3, 4 et 5.

**11.** Un micro-organisme selon la revendication 6, caractérisé en ce qu'il provient d'un micro-organisme tel que défini dans les revendications 7, 8 ou 9.

19